# EUROPÄISCHE PATENTANMELDUNG

(11) **EP 2 050 472 A1**
(43) Veröffentlichungstag der Anmeldung: **22.04.2009**
(21) Anmeldenummer: 08450159.2
(22) Anmeldetag: 16.10.2008
(51) Int. Cl.: A61L 9/12, A01M 1/20

(54) **Duftspender**

(30) Priorität: 17.10.2007 AT 16742007
(71) Anmelder: Granitzer, Christopher, 2340 Mödling (AT)
(72) Erfinder: Hoisl, Bernhard, 1020 Wien (AT); Granitzer, Christopher, 2340 Mödling (AT)
(74) Vertreter: Beer, Manfred

(57) **Zusammenfassung**

Es wird ein Duftspender beschrieben, der mehrere auswechselbare Duftbehälter (1) aufweist, die unterschiedlich riechende, Duftstoffe enthaltende, Flüssigkeiten enthalten und in einem Aufnahmeraum 2 des Gehäuses (6) aufgenommen sind. Im Gehäuse (6) ist wenigstens ein Ventilator (3) vorgesehen. Vor dem Ventilator (3) liegen Körper (4), die von der Duftstoffe enthaltenden Flüssigkeit benetzt werden und die bei Bedarf auch beheizbar sind bzw. in Schwingung versetzt werden können. Die Körper (4) nehmen die aus den Duftbehältern (1) über elektrisch angesteuerte Ventile (5), zum Beispiel Magnetventile oder Piezo Ventile, tretende, Duftstoffe enthaltende, Flüssigkeit auf. Durch den Luftstrom, der die Körper (4) überstreicht, werden Duftstoffe abgegeben. Die Stromversorgung wird wahlweise über einen USB Anschluss, eine Steckdose oder eine Batterie sichergestellt. Die elektronische Steuerungseinheit (7), zum Beispiel eine Speicher programmierbare Steuerung (SPS), ein mikroprozessorunterstützter elektronischer Regelkreis, befindet sich im Gehäuse (6) und steuert die elektrisch ansteuerbaren Ventile (5) sowie die Ventilatoren (3) an. Das Gerät besitzt die Möglichkeit Duftinformationen zu speichern, abzuändern und abzurufen. Die Erfindung ist über Computer, eine Fernsteuerung oder direkt bedienbar.

## Beschreibung

Die Erfindung betrifft einen Duftspender mit einem Gehäuse, in dem mehrere Aufnahmeräume für jeweils einen Duftstoff enthaltende Behälter vorgesehen sind, und mit wenigstens einem Ventilator zum Erzeugen eines Duftstoffe aus dem Gehäuse tragenden Luftstromes (EP 1 543 844 A).

Um einen Duft in die Umgebung (Luft) abzugeben, sind verschiedene Vorrichtungen und Verfahren bekannt.

So zeigt die DE 296 21 752 U eine Vorrichtung zum Verteilen von Duftstoffen, bei welcher ein Ventilator vorgesehen ist, der einen Luftstrom gegen eine Verteilermatte richtet, die mit einem Duftstoff enthaltenden Öl getränkt wird, wobei das Duftstoff enthaltende Öl mit Hilfe einer Solenoidpumpe aus einem Vorratstank durch Kanülen zu der Verteilermatte befördert wird.

Aus der EP 0 004 039 B ist ein Flüssigkeitszerstäuber bekannt, bei welchem die zu zerstäubende Flüssigkeit unter Ausnützen der Kapillarwirkung über einen Docht auf eine Zerstäuberplatte befördert wird. Die Zerstäuberplatte wird durch einen Schwingungsgenerator zu Schwingungen angeregt.

Nachteilig bei der aus der EP 0 004 039 B bekannten Vorrichtung ist die Verwendung kapillarer Kanülen, die zu Verstopfungen neigen.

Aus der DE 103 44 308 A ist ein Duftspender bekannt, der einen mit Duftstoffen gefüllten, mechanisch kippbaren Behälter umfasst. Die Duftstoffe sollen beim Kippen über eine Öffnung an einem den Duftstoff aufnehmenden Körper und in weiterer Folge durch einen von einem Ventilator erzeugten Luftstrom an die Umgebungsluft abgegeben werden. Nachteilig bei dieser Vorrichtung ist es, dass eine Pumpe erforderlich ist, die einen Druck erzeugt, um die Duftstoff enthaltende Flüssigkeit zu transportieren. Nachteilig bei der Vorrichtung gemäß der DE 103 44 308 U ist der große mechanische Aufwand, sowie der damit verbundene Bedarf an Platz für den Behälter und die regelungstechnischen Schwierigkeiten, die mit dieser Art des Aufbaues verbunden sind. Da der Füllstand des Behälters und der Kippwinkel im Bezug zueinander stehen, kann nur schwer eine geregelte Menge an Duftstoff abgegeben werden.

Die US 5,167,877 A zeigt einen Duftspender mit mehreren Behältern für verschiedene Duftstoffe. Es soll das Abgeben verschiedener Duftstoffe möglich sein. Hiezu wird Luft von einer Luftpumpe zu einem Verteiler gepumpt, in den Leitungen, die zu den Duftstoffbehältern führen, eingreifen. Die Duftstoffbehälter sind auf einem Drehtisch angeordnet. Luft soll abwechselnd in die Duftstoffbehälter gedrückt, und dabei auch gereinigt werden. Das Drehen des Tisches erfolgt mit Hilfe einer mit einem Elektromagneten betätigten Schubstange, die mit Nasen an der Unterseite des Tisches zusammenwirkt.

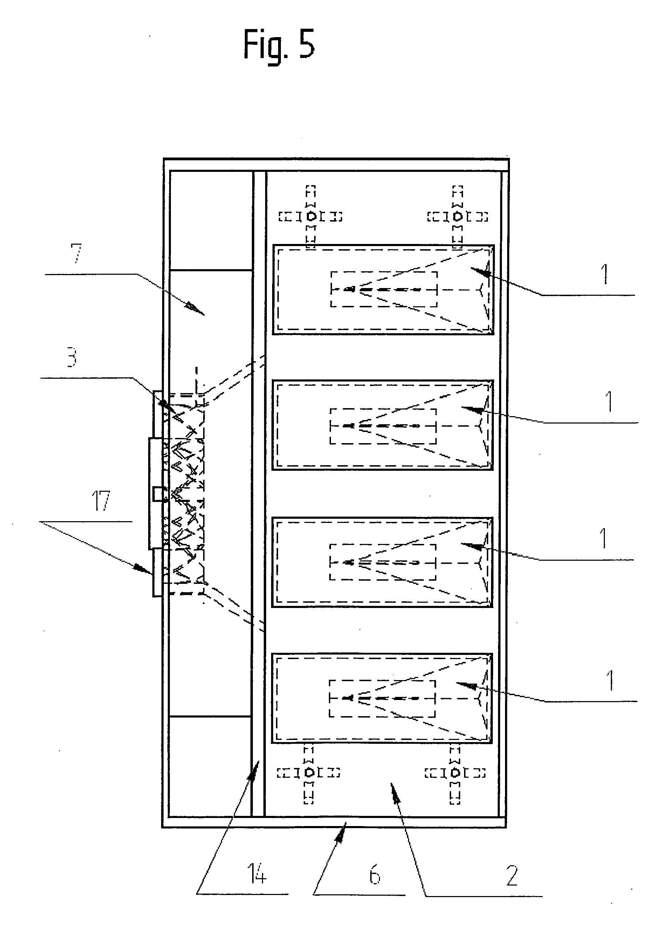

Aus der US 2006/0175426 A ist eine Duftstoffverteilvorrichtung mit Vorratsbehältern bekannt, wobei die Duftstoffverteilung durch einen Mikroprozessor gesteuert werden soll. Den austauschbaren Vorratsbehältern sollen elektromagnetische Duftstoffverteilvorrichtungen in Form von piezoelektrisch betätigten Sprühvorrichtungen zugeordnet sein (Fig. 5).

Die EP 1 543 844 A zeigt eine Vorrichtung zum Verteilen mehrerer Duftstoffe. In das Gerät (diffusor 20) wird ein Einsatz eingesetzt, der in einem Gehäuse einen Träger mit Aufnahmen für mehrere Duftvorratselemente aufweist. In dem Gerät ist ein Gebläse und eine Heizvorrichtung untergebracht. Der Träger wird gedreht, um das gewünschte Duftvorratselement in Stellung zu bringen. Es kann die Duftstoffabgabe programmiert werden.

Die WO 2006/0048891 A zeigt und beschreibt einen Duftspender mit einem Einsatz, in dem mehrere Duftstoffvorratsbehälter mit Dochten vorgesehen sind. Oberhalb des Einsatzes ist drehbar ein Duftstoffverteiler angeordnet, der durch Drehen wahlweise dem einen oder anderen Behälter 31 zugeordnet wird. Tröpfchen des Duftstoffes werden nach oben ausgestossen.

Der Erfindung liegt die Aufgabe zugrunde, einen Duftspender der eingangs genannten Gattung anzugeben, bei welchem eine problemlose Duftstoffabgabe möglich ist, wobei die Abgabe von Duftstoffen willkürlich geregelt werden kann.

Gelöst wird diese Aufgabe erfindungsgemäß mit einem Duftspender, welcher die Merkmale des unabhängigen Anspruch 1 aufweist.

Bevorzugte und vorteilhafte Ausgestaltungen der Erfindung sind Gegenstand der Unteransprüche.

Da bei dem Duftspender gemäß der Erfindung jedem der auswechselbaren Behälter ein Ventil zugeordnet ist, das in einer beispielhaften Ausführungsform von einer Steuerungseinheit nach einem vorgespeicherten Programm geöffnet und geschlossen werden kann, ist es möglich, die Abgabe der Duftstoffe weitgehend beliebig zu gestalten.

Mit Vorteil kann bei der Erfindung vorgesehen sein, dass zwischen dem Aufnahmeraum, in welchen bei der erfindungsgemäßen Vorrichtung der Behälter eingesetzt ist, und einer Zwischenwand das elektrisch angesteuerte Ventil vorgesehen ist.

Dabei kann erfindungsgemäß vorgesehen sein, dass in den Auslassöffnungen der Behälter (in deren Boden vorgesehen) selbsttätig wirkende Verschlussorgane vorgesehen sind, die verhindern, dass flüssige Duftstoffträger aus Behältern austreten, bevor diese in die erfindungsgemäßen Duftspender eingesetzt werden.

Bei der Erfindung ist in einer beispielhaften Ausführungsform mit Vorteil vorgesehen, dass unterhalb der Ventile, mit welchen die Abgabe von Duftstoffen aus den Behältern, die in dem Aufnahmeraum angeordnet sind, geregelt wird, ein Körper vorgesehen ist, der von einem durch einen Ventilator erzeugten Luftstrom bestrichen wird.

Bei der Erfindung besteht die Möglichkeit, unterschiedliche Düfte mit Hilfe des erfindungsgemäßen Duftspenders mit unterschiedlicher Intensität und Dauer abzugeben, indem die Ventile geregelt angesteuert werden und Duftstoffe an die unter den Behältern angeordneten Körper abgeben.

Es besteht auch die Möglichkeit, den Ventilator oder die Ventilatoren elektronisch angesteuert zu betreiben, wodurch die Möglichkeit geschaffen wird, Luftströmungen zu verändern und somit die Geschwindigkeit der Luft, welche über die Körper streicht, zu beeinflussen. Damit wird die Verteilung der Duftstoffe enthaltenden Flüssigkeit am Körper optimiert und die Duftabgabe beschleunigt.

In einer beispielhaften Ausführungsform der Erfindung ist vorgesehen, dass das jedem Behälter der Duftstoffe enthält zugeordnete Ventil auf der dem Behälter zugekehrten Seite einer Zwischenwand des Gehäuses erfindungsgemäßen Duftspenders angeordnet ist, wobei die Zwischenwand den Boden des Aufnahmeraumes für die Behälter bildet. Die Körper befinden sich bei dieser Ausführungsform unterhalb der Ventile und der Zwischenwand des Gehäuses, wobei die Möglichkeit besteht, einen Ventilator für alle Körper, oder jedem Körper einen eigenen Ventilator zuzuordnen.

Der erfindungsgemäße Duftspender erlaubt es, Düfte (Gerüche) voneinander unabhängig und entsprechend der Betätigung der Ventile dynamisch abzugeben.

Die Gerüche (Duftstoffe) können voneinander unabhängig und entsprechend der Schaltzeit der Ventile dynamisch abgegeben werden. Vergleichbar ist dieser Vorgang mit einem Musikstück, das aus dem Takt und den Noten - in diesem Fall den Duftnoten - besteht. Durch den ständigen Wechsel der Düfte ermüdet das Geruchsorgan nicht und das erfindungsgemäße Gerät ist auch für Aromatherapien und Geruchsstimulation im Eigenheim einsetzbar (zum Beispiel: Raumduftspender, Geruch unterstützte Computerspiele, E-Commerce Entspannungsseiten im Internet, Duftdesigner für Parfums). Der Wirkungsbereich der Düfte kann über die abgegebene Menge, sowie über die Ansteuerung des Ventilators oder der Ventilatoren beeinflusst werden. Die Drehzahl des Ventilators bestimmt den Luftstrom und somit auch die Anzahl der Duftpartikel in dem abgegebenen Luft-Duftstoff-Gemisch. Durch diesen Aufbau können unzählige, das Riechorgan ansprechende Gerüche modelliert werden, wobei es vorteilhaft ist, Grundkonfigurationen der Gerüche vorzuschlagen, die der Benutzer bei Bedarf durch die Steuerung abändern kann oder selbst neu definiert und speichert.

Die Duftstoffe enthaltenden Flüssigkeiten werden in auswechselbaren Behältern, die nach oben hin offen und dort beispielsweise durch eine Membran geschlossen sind, dem Ventil bereitgestellt, wodurch eine Flüssigkeitsabgabe an das Ventil unter atmosphärischem Druck möglich wird. Die Abgabe der Flüssigkeit an die Körper wird durch die elektrisch ansteuerbaren Ventile mit kurzen Schaltzeiten (∼ 20 ms) moduliert.

Eine von jedem Körper ausgehende und in das jeweils zugeordnete Ventil eingreifende Spitze nimmt bei einer beispielhaften und bevorzugten Ausführungsform der Erfindung den Tropfen von mit Duftstoffen beladener Flüssigkeit vom Ventil schnell und gezielt auf, und gibt diesen an den Körper weiter. Dabei werden die Adhäsionskräfte der Spitze zum Tropfen genutzt.

Der Körper besitzt beispielsweise eine vergrößerte Oberfläche, welche die Aufnahme der Duftstoffe, ebenfalls durch Adhäsionskräfte, begünstigt und über die Oberfläche die Duftstoffe unter Hilfe des Ventilators zusätzlich verteilt und abgibt. Der Körper kann auch, sollte es der Verteilung und Abgabe des Duftstoffes dienlich sein, beheizt oder in Schwingung versetzt werden.

Um maximalen Komfort der Bedienung zu gewährleisten, ist der Duftspender über Computer, eine Fernsteuerung oder direkt bedienbar.

Ein Reinigen des Gerätes ist durch separate Reinigungslösungen in speziell vorgesehenen wechselbaren Behältern möglich.

Weitere Einzelheiten des erfindungsgemäßen Duftspenders werden beispielhaft mit Bezug auf die angeschlossenen Zeichnungen, die auf wichtigste mechanische Teile begrenzt sind, erläutert.

Es zeigt:

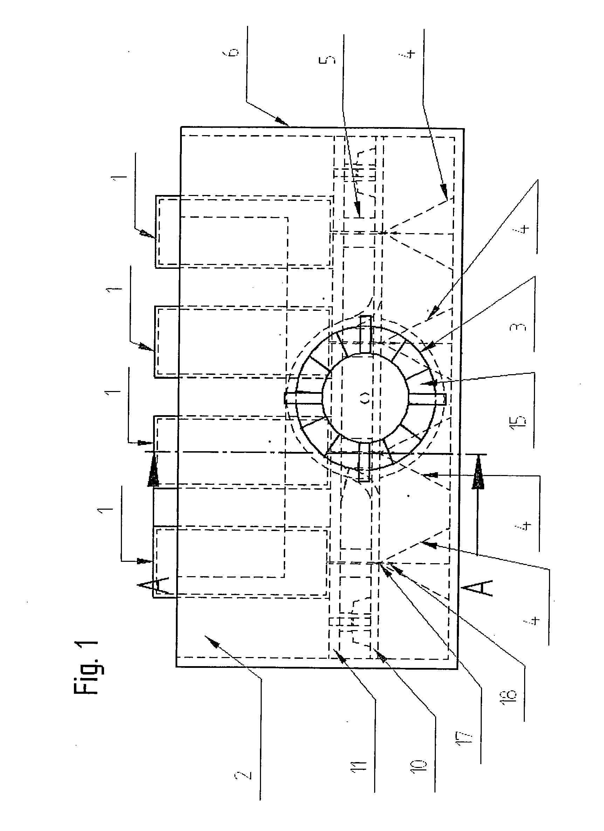

Fig. 1 einen Längsschnitt,
Fig. 2 einen Querschnitt längs der Linie A-A in Fig. 1,
Fig. 3 ein Detail B der Spitze im Flüssigkeitskanal zu der Öffnung des Ventils,

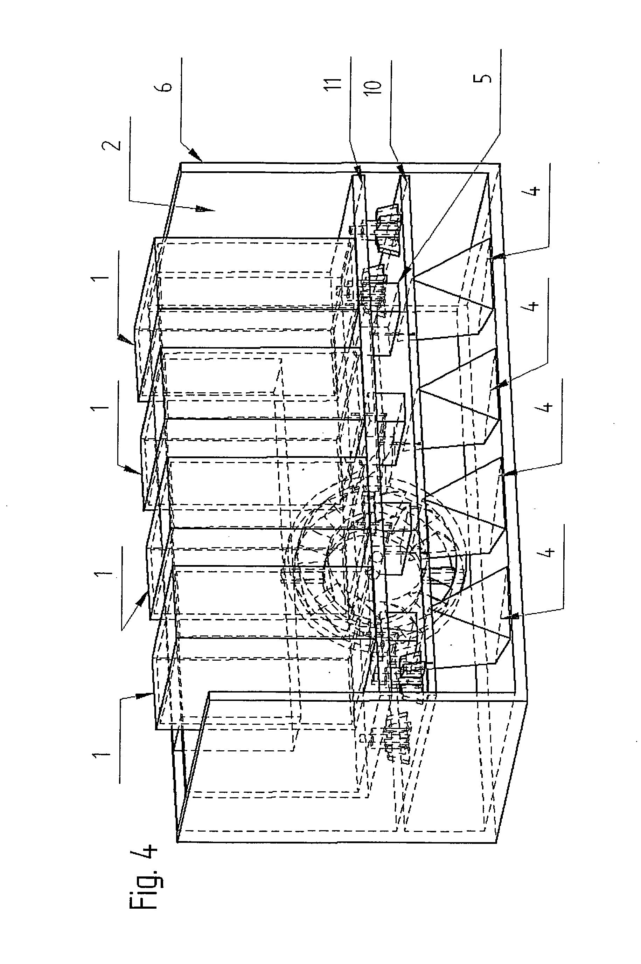

Fig. 4 eine perspektivische Ansicht des Duftspenders der Erfindung und
Fig. 5 eine Draufsicht auf den Duftspender.

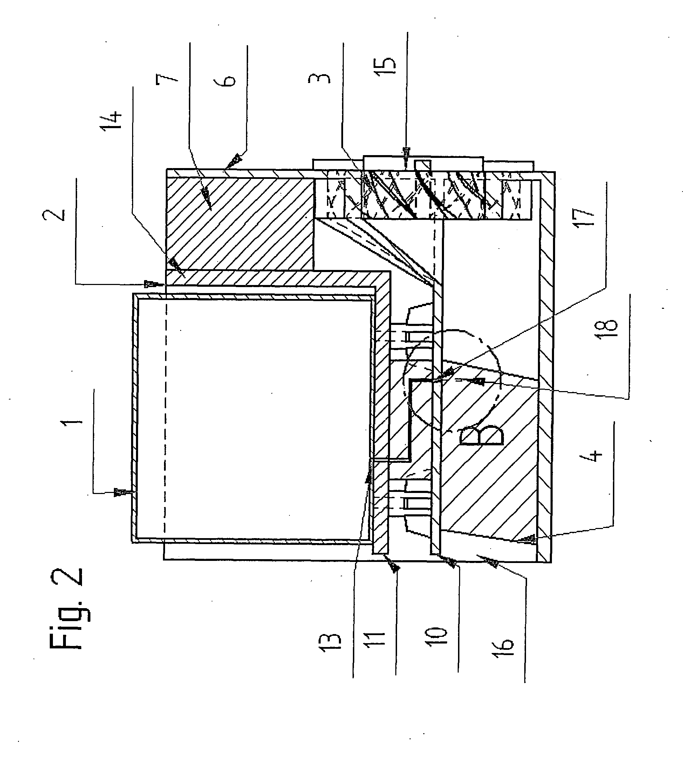

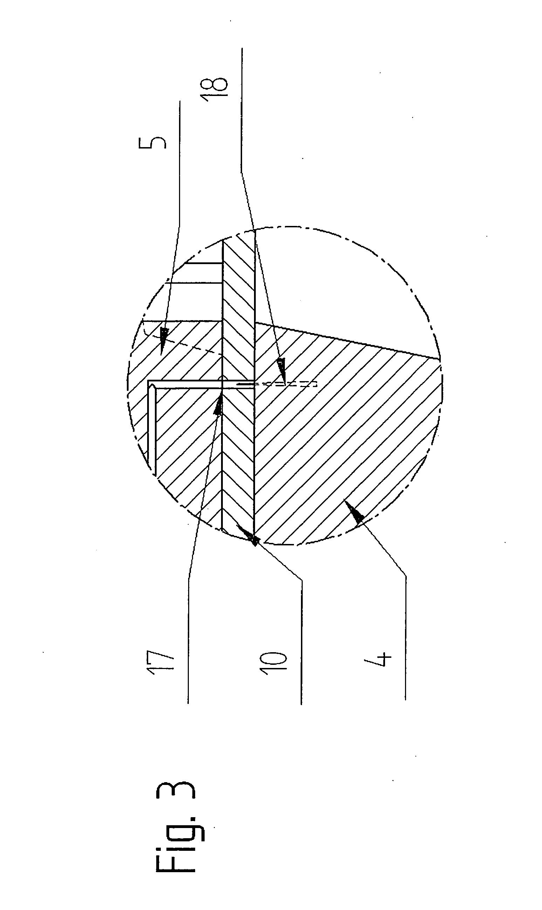

Wie beispielhaft in Fig. 2 ersichtlich, wird wenigstens ein auswechselbarer Behälter 1, in dem sich wenigstens einen Duftstoff enthaltende Flüssigkeit befindet, in einen Aufnahmeraum 2 im Gehäuse 6 des Duftspenders aufgenommen. Der Boden 11 des Aufnahmeraumes 2 besitzt Durchgänge, über die der Ventileinlass 13 eines von mehreren Ventilen 5 mit dem Behälter 1 verbunden wird. Für jeden Behälter 1 ist ein Ventil 5 vorgesehen, das in der gezeigten Ausführungsform zwischen dem Boden 11 des Aufnahmeraumes 2 und einer Zwischenwand 10 des Gehäuses 6 angeordnet ist. Die Ventile 5, die beispielsweise elektronisch steuerbare Ventile (5) sind, werden durch eine Speicher programmierbare Steuerungseinheit 7 angesprochen. Bei geöffnetem Ventil 5 findet ein Flüssigkeitstransport vom Ventil 5 zu dem Körper 4 über einen Durchgang in der Zwischenwand 10 des Gehäuses 6 statt. Eine Spitze 18 (dargestellt in Detailansicht B in Fig. 3) als Teil des keilförmigen Körpers 4 greift in den Auslasskanal 17 des Ventils 5 ein und nimmt Duftstoff enthaltende Flüssigkeit, insbesondere in Form von Tropfen, vom Ventil 5 schnell und gezielt auf, um diesen an den Körper 4 optimal weiterzugeben. Dabei werden die Adhäsionskräfte der Spitze 18 zum Tropfen der Flüssigkeit genutzt. Die Spitze 18 muss nicht in den Auslasskanal 17 eingreifen, es genügt, wenn das freie Ende der Spitze 18 dem Auslasskanal so zugeordnet ist, dass sie Duftstoffe enthaltende Flüssigkeit, insbesondere Tropfen der Duftstoffe enthaltenden Flüssigkeit, sicher übernehmen und an den Körper 4 weiterleiten kann.

Das Gehäuse 6 ist so konstruiert, dass die elektronische Speicher programmierbare Steuerungseinrichtung 7 von den Flüssigkeiten in den Behältern 1 sicher getrennt ist, beispielsweise durch eine Rückwand 14 des Aufnahmeraumes 2.

Das Gehäuse 6 bildet zudem unterhalb der Zwischenwand 10 eine Düse, durch die über einen Lufteinlass 15 vom dem wenigstens einen Ventilator 3 angesaugte Luft zu einem Luftauslass 16 strömt. Dabei nimmt die Luft den auf den Körper 4 aufgetragenen Duftstoff auf und gibt das Gemisch an die Luft ab. Die Körper 4 weisen dabei eine große Oberfläche auf, welche die Aufnahme des Duftes unterstützt.

Weiters kann die Duftabgabe durch Erhöhen der Temperatur des Körpers 4 begünstigt werden.

Gegebenenfalls kann das Verteilen und Abgeben des Duftstoffes auf den Körper 4 begünstigt werden, indem dieser durch einen Schwingungserzeuger in Schwingung versetzt wird.

Zusammenfassend kann ein Ausführungsbeispiel der Erfindung wie folgt beschrieben werden:

Es wird ein Duftspender beschrieben, der mehrere auswechselbare Duftbehälter 1 aufweist, die unterschiedlich riechende, Duftstoffe enthaltende, Flüssigkeiten enthalten und in einem Aufnahmeraum 2 des Gehäuses 6 aufgenommen sind. Im Gehäuse 6 ist wenigstens ein Ventilator 3 vorgesehen. Vor dem Ventilator 3 liegen Körper 4, die von der Duftstoffe enthaltenden Flüssigkeit benetzt werden und die bei Bedarf auch beheizbar sind bzw. in Schwingung versetzt werden können. Die Körper 4 nehmen die aus den Duftbehältern 1 über elektrisch angesteuerte Ventile 5, zum Beispiel Magnetventile oder Piezo Ventile, tretende, Duftstoffe enthaltende, Flüssigkeit auf. Durch den Luftstrom, der die Körper 4 überstreicht, werden Duftstoffe abgegeben. Die Stromversorgung wird wahlweise über einen USB Anschluss, eine Steckdose oder eine Batterie sichergestellt. Die elektronische Steuerungseinheit 7, zum Beispiel eine Speicher programmierbare Steuerung (SPS), ein mikroprozessorunterstützter elektronischer Regelkreis, befindet sich im Gehäuse 6 und steuert die elektrisch ansteuerbaren Ventile 5 sowie die Ventilatoren 3 an. Das Gerät besitzt die Möglichkeit Duftinformationen zu speichern, abzuändern und abzurufen. Die Erfindung ist über Computer, eine Fernsteuerung oder direkt bedienbar.

## Patentansprüche

1. Duftspender mit einem Gehäuse (6), in dem wenigstens ein Aufnahmeraum (2) für Duftstoff enthaltende Flüssigkeit enthaltende Behälter (1) vorgesehen sind, und mit wenigstens einem Ventilator (3) zum Erzeugen eines Duftstoffe aus dem Gehäuse (6) tragenden Luftstromes, **dadurch gekennzeichnet, dass** dem Auslass von jedem der Behälter (1) ein elektrisch von einer programmierbaren Steuerungseinheit (7) gesteuertes Ventil (5) zugeordnet ist, und dass unter jedem Behälter (1) ein Körper (4) vorgesehen ist, der von dem vom Ventilator (3) erzeugten Luftstrom bestrichen wird.

2. Duftspender nach Anspruch 1, **dadurch gekennzeichnet, dass** die Körper (4) sich vom Behälter (1) bzw. dem Ventil (5) weg keilförmig verbreiternd ausgebildet ist.

3. Duftspender nach Anspruch 1 oder 2, **dadurch gekennzeichnet, dass** der Aufnahmeraum (2) für Behälter (1) oberhalb einer Zwischenwand (10) des Gehäuses (6) angeordnet ist.

4. Duftspender nach Anspruch 3, **dadurch gekennzeichnet, dass** das Ventil (5) auf der dem Behälter (1) zugewendeten Seite der Zwischenwand (10) des Gehäuses (6) und unter dem Boden (11) des Aufnahmeraumes (2) angeordnet ist.

5. Duftspender nach einem der Ansprüche 1 bis 4, **dadurch gekennzeichnet, dass** der Ventilator (3), der einen den Körper (4) bestreichenden Luftstrom erzeugt, unterhalb des Aufnahmeraumes (2) angeordnet ist.

6. Duftspender nach einem der Ansprüche 1 bis 5, **dadurch gekennzeichnet, dass** dem Körper (4) ein Schwingungserzeuger zugeordnet ist.

7. Duftspender nach einem der Ansprüche 1 bis 6, **dadurch gekennzeichnet, dass** dem Körper (4) eine Heizung zugeordnet ist.

8. Duftspender nach einem der Ansprüche 1 bis 7, **dadurch gekennzeichnet, dass** der Aufnahmeraum (2) ebenso wie das Gehäuse (6) im Bereich unterhalb des Aufnahmerraumes (2) bzw. der Zwischenwand (10) auf der dem wenigstens einem Ventilator (3) gegenüberliegenden Seite durch einen Luftauslass (16) offen ist.

9. Duftspender nach einem der Ansprüche 1 bis 8, **dadurch gekennzeichnet, dass** jeder Körper (4) eine Spitze (18) aufweist, die in die Auslassöffnung (17) der dem Behälter (1) zugeordneten Ventile (5) eingreift.

10. Duftspender nach einem der Ansprüche 1 bis 9, **dadurch gekennzeichnet, dass** die Behälter (1) an ihrer in der Gebrauchslage oben zu liegen kommenden Seite durch eine Membran verschlossen sind.

11. Duftspender nach einem der Ansprüche 1 bis 10, **dadurch gekennzeichnet, dass** der Auslassöffnung der Behälter (1) ein selbsttätig verschließendes Verschlussorgan zugeordnet ist.

12. Duftspender nach einem der Ansprüche 1 bis 11, **dadurch gekennzeichnet, dass** der Bereich des Gehäuses (6) unterhalb der Zwischenwand (10) in Kammern unterteilt ist, und dass in jeder der unterhalb der Zwischenwand (10) vorgesehenen Kammern ein Ventilator (3) zugeordnet ist.

13. Duftspender nach einem der Ansprüche 8 bis 13, **dadurch gekennzeichnet, dass** die Spitze (18) und der Körper (4) aus einem durch die Duftstoff enthaltende Flüssigkeit benetzbaren Werkstoff bestehen.
